# EUROPÄISCHE PATENTSCHRIFT

(11) **EP 2 265 294 B1**
(45) Veröffentlichungstag und Bekanntmachung des Hinweises auf die Patenterteilung: **12.10.2011**
(21) Anmeldenummer: 09721521.4
(22) Anmeldetag: 16.03.2009
(51) Int. Cl.: A61L 29/08, A61L 31/10

(54) **MEDIZINISCHE GERÄTE MIT HYDROPHILEN BESCHICHTUNGEN**
MEDICAL DEVICE HAVING HYDROPHILIC COATINGS
INSTRUMENTS MÉDICAUX COMPORTANT DES REVÊTEMENTS HYDROPHILES

(30) Priorität: 20.03.2008 DE 102008153057
(43) Veröffentlichungstag der Anmeldung: 29.12.2010
(73) Patentinhaber: Bayer MaterialScience AG, 51368 Leverkusen (DE)
(72) Erfinder: KÖCHER, Jürgen, 40764 Langenfeld (DE); RISCHE, Thorsten, Columbus, Georgia, 31909 (US)
(86) Internationale Anmeldenummer: PCT/EP2009/001901
(87) Internationale Veröffentlichungsnummer: WO 2009/115266

(56) Entgegenhaltungen:
- WO-A-2006/037321
- WO-A-2006/101573
- JP-A- 2002 128 859
- US-A- 5 041 100
- US-A1- 2005 182 188

## Beschreibung

Die vorliegende Erfindung betrifft medizinische Geräte mit hydrophilen und blutverträglichen Beschichtungen aus Polyurethanharnstoffen. Diese medizinischen Geräte mit verbesserter Oberflächenbeschaffenheit bieten aufgrund einer geringeren Reibung und durch deren Fähigkeit, bei Kontakt mit Blut die Gefahr von Blutgerinnseln zu reduzieren, Vorteile bei der Anwendung.

Die Benutzung von medizinischen Geräten, beispielsweise von Kathetern, kann durch die Ausrüstung mit hydrophilen Oberflächen stark verbessert werden. Das Einsetzen und Verschieben von Urin- oder Blutgefäßkathetern wird dadurch einfacher, dass hydrophile Oberflächen im Kontakt mit Blut oder Urin einen Wasserfilm adsorbieren. Hierdurch wird die Reibung der Katheteroberfläche gegenüber den Gefäßwänden verringert, so dass sich der Katheter leichter einsetzen und bewegen lässt. Auch eine direkte Wässerung der Geräte vor dem Eingriff kann vorgenommen werden, um durch die Bildung eines homogenen Wasserfilms die Reibung zu vermindern. Die betroffenen Patienten haben weniger Schmerzen, und das Risiko von Verletzungen der Gefäßwände wird dadurch reduziert. Darüber hinaus besteht bei der Anwendung von Kathetern im Blutkontakt immer die Gefahr, dass sich Blutgerinnsel bilden. In diesem Kontext werden im Allgemeinen hydrophile Beschichtungen als hilfreich für antithrombogene Beschichtungen angesehen.

Katheter mit hydrophil ausgerüsteten Oberflächen sind aus dem Stand der Technik an sich bekannt.

So beschreibt beispielsweise die WO 99/38545 A1 Katheter, die in einer ersten Ausführungsform aus einer Grundbeschichtung und einer gleitfähigen hydrophilen Beschichtung bestehen. Ferner wird in diesem Stand der Technik auch eine Ausführungsform beschrieben, in welcher nur eine gleitfähige Beschichtung, d.h. ein Beschichtungssystem ohne Grundbeschichtung, verwendet wird. In diesem Fall wird eine gleitfähige Beschichtung aus einem Polyurethan verwendet. Dabei wird das Isocyanat als Bindeglied auf der Oberfläche zur Anknüpfung hydrophiler Gruppen genutzt. Auf dem medizinischen Gerät sind somit toxische Isocyanate vorgesehen, und es muss zur Beschleunigung der Aushärtung auf sehr toxische zinnhaltige Katalysatoren zurückgegriffen werden.

Aus der WO 2006/037321 A1 sind medizinische Geräte mit einer angefeuchteten hydrophilen Oberfläche bekannt, welche die Gleiteigenschaften des Gerätes verbessern soll. Die Oberfläche wird gebildet durch eine Beschichtungszusammensetzung mit einem hydrophilen Polymer und einem Befeuchtungsmittel, umfassend Wasser und mindestens ein Gleitmittel. Die aus diesem Stand der Technik bekannte Beschichtungszusammensetzung besteht aus mehreren Bestandteilen, die allesamt funktional zusammenarbeiten müssen, um die resultierende Beschichtung mit den gewünschten Eigenschaften zu versehen.

Aus der US 2003/0203991 A1 sind hydrophile Beschichtungsmaterialien bekannt, die auf Mischungen von hydrophoben mit hydrophilen Polymeren basieren. Entsprechende Beschichtungszusammensetzungen für medizinische Geräte umfassen (a) eine wässrige polymere Matrix; (b) ein hydrophiles Polymer; (c) ein kolloidales Metalloxid und (d) ein Vernetzer. Die erforderliche Hydrophilie der Beschichtung gemäß US 2003/0203991 A1 wird durch das Polymer (b) erreicht, welches in die entsprechende polymere Matrix eingearbeitet ist. Als polymere Matrix, nicht jedoch als hydrophiles Polymer, werden unter anderem auch Polyurethandispersionen verwendet. Die umfangreiche ionische Modifizierung dieser Polyurethandispersionen kann zu einer unerwünschten Reduktion der Hydrophilie führen.

Mischungen aus Polyurethan und Polyvinylpyrrolidon als der die Hydrophilie bewirkende Bestandteil werden ferner in US 5,061,424 beschrieben. Darüber hinaus beschreiben US 5,041,100 und US 2005/054774 A1 jeweils polyurethanhaltige Beschichtungszusammensetzungen mit Polyethylenoxid (US 5,041,100) bzw. Acrylaten (US 2005/054774) als die die Hydrophilie bewirkenden Bestandteile.

US 2006/040253 A1 beschreibt hydrophile Beschichtung von medizinischen Geräten zur Verbesserung der Gleiteigenschaften, wobei die Zusammensetzung zumindest ein wasserlösliches gleitfähiges Polymer und ein unlösliches Polymer aufweist. Das wasserlösliche gleitfähige Polymer wird unter anderem ausgewählt aus der Gruppe, bestehend aus Polyethylenoxid, Polypropylenoxid, Polyethylvinylalkohol, Polyethylvinylacetat und Polyvinylpyrrolidon, während das unlösliches Polymer unter anderem durch Polyurethane, Polyesterurethane und Polyetherurethane gebildet wird.

Aliphatische Polyetherpolyurethane für hydrophile Beschichtungen sind ebenfalls kommerziell erhältlich, so zum Beispiel das Tecogel^{®} (Thermedics Polymer Products) oder das Hydroslip^{®} (CardioTech International Inc.).

Sowohl die in der Literatur beschriebenen Mischungen als auch die kommerziell erhältlichen Polyetherpolyurethane weisen verschiedene Nachteile auf. So handelt es sich bei diesen Mischungen um Mehrkomponentensysteme, d.h. sie umfassen mehrere separate Beschichtungen, und sind daher aufwändig in der Herstellung, insbesondere auch solche Systeme, die durch kovalente Verknüpfung zweier Polymere aufgebaut werden (vgl. US 2003/0203991 A1). Die aliphatischen Polyetherurethane sind von der Anwendung her einfacher, lassen sich aber häufig nur mit Anteilen von organischen Lösungsmitteln verarbeiten. Dieses ist aber bei Anwendung von medizinischen Geräten insbesondere in menschlichen oder tierischen Körpern aufgrund der Gefahr der Freisetzung von Lösemittelresten aus den Beschichtungen unerwünscht. Daher besteht grundsätzlich weiter Bedarf an medizinischen Geräten zur Verwendung innerhalb des menschlichen oder tierischen Körpers, welche hydrophile Oberflächen aufweisen, und vorzugsweise die aufgezeigten Nachteile des Standes der Technik beheben.

In diesem Kontext empfiehlt die US 5,589,563 oberflächenmodifizierte Endgruppen für biomedizinisch Polymere, welche zur Beschichtung von medizinischen Geräten verwendet werden können. Diese Polymere umfassen unterschiedliche Endgruppen, welche ausgewählt werden aus Aminen, fluorierten Alkanolen, Polydimethylsiloxanen und aminterminierten Polyethylenoxiden. Diese Polymere weisen jedoch als Beschichtung für medizinische Geräte keine zufriedenstellenden Eigenschaften, insbesondere im Hinblick auf die erforderliche Hydrophilie, auf.

Die Aufgabe der vorliegenden Erfindung ist somit die Bereitstellung von medizinischen Geräten mit hydrophilen Oberflächen. Da diese Oberflächen häufig im Blutkontakt eingesetzt werden, sollen die Oberflächen dieser Materialien auch eine gute Blutverträglichkeit besitzen und insbesondere die Gefahr der Bildung von Blutgerinnseln reduzieren.

Gegenstand dieser Erfindung sind medizinische Geräte mit hydrophilen Oberflächen, die durch Beschichtung mit speziellen Polyurethandispersionen erzeugt werden können.

Die erfindungsgemäßen medizinischen Geräte umfassen mindestens eine Beschichtung, enthaltend mindestens einen Polyurethanharnstoff, der mit einer Copolymereinheit aus Polyethylenoxid und Polypropylenoxid terminiert ist.

Erfindungsgemäß wurde herausgefunden, dass sich Zusammensetzungen aus diesen spezifischen Polyurethanharnstoffen hervorragend als Beschichtungen von medizinischen Geräten eignen, diese mit einer hervorragenden gleitfähigen Beschichtung versehen und gleichzeitig die Gefahr der Bildung von Blutgerinnseln während der Behandlung mit dem medizinischen Gerät reduzieren.

Polyurethanharnstoffe im Sinne der vorliegenden Erfindung sind polymere Verbindungen, die
(a) mindestens zwei Urethangruppen enthaltende Wiederholungseinheiten der folgenden allgemeinen Struktur 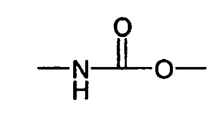 und
   mindestens eine Harnstoffgruppen enthaltende Wiederholungseinheit 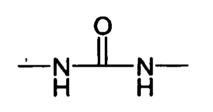 aufweisen.

Die erfindungsgemäß zu verwendenden Beschichtungszusammensetzungen basieren auf Polyurethanharnstoffen, welche im Wesentlichen keine ionische Modifizierung aufweisen. Hierunter wird im Rahmen der vorliegenden Erfindung verstanden, dass die erfindungsgemäß zu verwendenden Polyurethanharnstoffe im Wesentlichen keine ionischen Gruppen, wie insbesondere keine Sulfonat-, Carboxylat-, Phosphat- und Phosphonatgruppen, aufweisen.

Unter dem Begriff "im Wesentlichen keine ionische Modifizierung" wird im Rahmen der vorliegenden Erfindung verstanden, dass eine ionische Modifizierung höchstens in einem Anteil von 2,50 Gew.-%, vorzugsweise höchstens 2,00 Gew.-%, insbesondere höchstens 1,50 Gew.-%, besonders bevorzugt höchstens 1,00 Gew.-%, speziell höchstens 0,50 Gew.-%, vorliegt, wobei es am bevorzugtsten ist, wenn überhaupt keine ionische Modifizierung des erfindungsgemäß vorgesehenen Polyurethanharnstoffs vorliegt.

Die erfindungsgemäß zur Beschichtung der medizinischen Geräte vorgesehenen Polyurethanharnstoffe sind bevorzugt im Wesentlichen lineare Moleküle, können jedoch auch verzweigt sein, was jedoch weniger bevorzugt ist. Unter im Wesentlichen linearen Molekülen versteht man leicht anvernetzte Systeme, die ein Polycarbonatpolyol mit einer mittleren Hydroxylfunktionalität von vorzugsweise 1,7 bis 2,3, insbesondere 1,8 bis 2,2, besonders bevorzugt 1,9 bis 2,1, aufweisen. Derartige Systeme lassen sich noch in einem ausreichenden Maß dispergieren.

Das zahlenmittlere Molekulargewicht der erfindungsgemäß bevorzugt verwendeten Polyurethanharnstoffe beträgt vorzugsweise 1000 bis 200000, besonders bevorzugt von 5000 bis 100000. Dabei wird das zahlenmittlere Molekulargewicht gegen Polystyrol als Standard in Dimethylacetamid bei 30 °C gemessen.

Die mittlere Teilchengröße der erfindungsgemäßen dispergierten Polyurethanharnstoffe beträgt vorzugsweise 10 bis 1000 nm, besonders bevorzugt 20 bis 800 nm, ganz besonders bevorzugt 50 bis 600 nm.

### Polyurethanharnstoffe

Im Folgenden werden die erfindungsgemäß zu verwendenden Beschichtungssysteme auf Basis von Polyurethanharnstoffen näher beschrieben.

Die erfindungsgemäß in den Beschichtungen von medizinischen Geräten verwendeten Polyurethanharnstoffe werden durch Umsetzung von Aufbaukomponenten hergestellt, die mindestens eine Polycarbonatpolyolkomponente, mindestens eine Polyisoyanatkomponente, mindestens eine Polyoxyalkylenetherkomponente, mindestens eine Diamin- und/oder Aminoalkoholkomponente und gegebenenfalls eine Polyolkomponente umfassen.

Im folgenden werden nun die einzelnen Aufbaukomponenten näher beschrieben.

### (a) Polycarbonatpolyol

Die Zusammensetzung der erfindungsgemäß vorgesehene Beschichtung eines Polyurethanharnstoffes umfasst Einheiten, welche auf mindestens ein Hydroxylgruppen enthaltendes Polycarbonat zurückgehen (Polycarbonatpolyol).

Grundsätzlich geeignet für das Einführen von Einheiten auf Basis eines Hydroxylgruppen enthaltenden Polycarbonats sind Polycarbonatpolyole, d. h. Polyhydroxyverbindungen, mit einer mittleren Hydroxylfunktionalität von 1,7 bis 2,3, vorzugsweise von 1,8 bis 2,2, besonders bevorzugt von 1,9 bis 2,1. Das Polycarbonat ist somit vorzugsweise im Wesentlichen linear ausgebildet und weist nur eine geringfügige dreidimensionale Vernetzung auf.

Als Hydroxylgruppen aufweisende Polycarbonate kommen Polycarbonate des Molekulargewichts (über die OH-Zahl bestimmte Molekulargewicht; DIN 53240) von vorzugsweise 400 bis 6000 g/mol, besonders bevorzugt 500 bis 5000 g/mol, insbesondere von 600 bis 3000 g/mol in Frage, die beispielsweise durch Reaktion von Kohlensäurederivaten, wie Diphenylcarbonat, Dimethylcarbonat oder Phosgen, mit Polyolen, vorzugsweise Diolen, erhältlich sind. Als derartige Diole kommen beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, Tetrabrombisphenol A aber auch Lacton-modifizierte Diole in Frage.

Bevorzugt enthält die Diolkomponente 40 bis 100 Gew.-% Hexandiol, bevorzugt 1,6-Hexandiol und/oder Hexandiol-Derivate, vorzugsweise solche, die neben endständigen OH-Gruppen Ether- oder Estergruppen aufweisen, z.B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten wurden. Auch Polyether-Polycarbonatdiole können eingesetzt werden. Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate auf Basis von Hexandiol-1,6, sowie modifizierend wirkenden Co-Diolen wie z. B. Butandiol-1,4 oder auch von ε-Caprolacton. Weitere bevorzugte Polycarbonatdiole sind solche auf Basis von Mischungen aus Hexandiol-1,6 und Butandiol-1,4.

### (b) Polyisocyanat

Die Zusammensetzung der erfindungsgemäß vorgesehene Beschichtung eines Polyurethanharnstoffes weist Einheiten auf, welche auf mindestens ein Polyisocyanat zurückgehen.

Als Polyisocyanate (b) können alle dem Fachmann bekannten aromatischen, araliphatischen, aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgenfreien Verfahren hergestellt wurden. Diese können auch Iminooxadiazindion-, Isocyanurat-, Uretdion-, Urethan-, Allophanat-, Biuret-, Harnstoff-, Oxadiazintrion, Oxazolidinon-, Acylharnstoff- und/oder Carbodiimid-Strukturen aufweisen. Die Polyisocyanate können einzeln oder in beliebigen Mischungen untereinander eingesetzt werden.

Bevorzugt werden Isocyanate aus der Reihe der aliphatischen oder cycloaliphatischen Vertreter eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente (b) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodecantriisocyanate, 1,3- sowie 1,4-Bis-(isocyanatomethyl)cyclohexane (H₆XDI), 3-Isocyanatomethyl-3,5,5-trimethylcyclohexylisocyanat (isophorondiisocyanat, IPDI), Bis-(4-isocyanatocyclohexyl)methan (H₁₂MDI) oder Bis-(isocyanatomethyl)norbornan (NBDI).

Ganz besonders bevorzugte Verbindungen der Komponente (b) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)norbornan (NBDI), 3(4)-Isocyanatomethyl-1-methylcyclohexylisocyanat (IMCI) und/oder 4,4'-Bis(isocyanatocyclohexyl)methan (H₁₂MDI) oder Gemische dieser Isocyanate. Weitere Beispiele sind Derivate aus den vorstehenden Diisocyanaten mit Uretdion-, Isocyanurat-, Urethan-, Allophanat-, Biuret-, Iminooxadiazindion- und/oder Oxadiazintrionstruktur mit mehr als zwei NCO-Gruppen.

Die Menge an Bestandteil (b) in der erfindungsgemäß zu verwendenden Beschichtungszusammensetzung beträgt vorzugsweise 1,0 bis 4,0 mol, besonders bevorzugt 1,2 bis 3,8 mol, insbesondere 1,5 bis 3,5 mol, jeweils bezogen auf den Bestandteil (a) der erfindungsgemäß zu verwendenden Beschichtungszusammensetzung.

### (c) Polyoxyalkylenether

Der in der vorliegenden Erfindung verwendete Polyurethanharnstoff weist Einheiten auf, welche auf ein Copolymer aus Polyethylenoxid und Polypropylenoxid zurückgehen. Diese Copolymereinheiten liegen als Endgruppen in dem Polyurethanharnstoff vor.

Nichtionisch hydrophilierende Verbindungen (c) sind beispielsweise einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Die Alkylenoxide Ethylenoxid und Propylenoxid können in beliebiger Reihenfolge oder auch im Gemisch bei der Alkoxylierungsreaktion eingesetzt werden können.

Bei den Polyalkylenoxidpolyetheralkoholen handelt es sich um gemischte Polyalkylenoxidpolyether aus Ethylenoxid und Propylenoxid, deren Alkylenoxideinheiten vorzugsweise zu mindestens 30 mol%, besonders bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol% Ethylenoxid- und maximal 60 mol-% Propylenoxideinheiten aufweisen.

Das mittlere Molgewicht des Polyoxyalkylenethers beträgt vorzugsweise 500 g/mol bis 5000 g/mol, besonders bevorzugt 1000 g/mol bis 4000 g/mol, insbesondere 1000 bis 3000 g/mol.

Die Menge an Bestandteil (c) in der erfindungsgemäß zu verwendenden Beschichtungszusammensetzung beträgt vorzugsweise 0,01 bis 0,5 mol, besonders bevorzugt 0,02 bis 0,4 mol, insbesondere 0,04 bis 0,3 mol, jeweils bezogen auf den Bestandteil (a) der erfindungsgemäß zu verwendenden Beschichtungszusammensetzung.

Erfindungsgemäß konnte gezeigt werden, dass sich die Polyurethanharnstoffe mit Endgruppen, die auf gemischten Polyoxyalkylenether aus Polyethylenoxid und Polypropylenoxid basieren, insbesondere dazu eignen, Beschichtungen mit einer hohen Hydrophilie zu erzeugen. Wie weiter unten im Vergleich zu Polyurethanharnstoffen, welche nur durch Polyethylenoxid terminiert sind, gezeigt wird, bewirken die erfindungsgemäßen Beschichtungen einen deutlich geringen Kontaktwinkel und sind somit hydrophiler ausgebildet.

### (d) Diamin oder Aminoalkohol

Die Zusammensetzung der erfindungsgemäß vorgesehenen Beschichtung eines Polyurethanharnstoffes weist Einheiten auf, welche auf mindestens ein Diamin oder ein Aminoalkohol zurückgehen.

Zur Herstellung der erfindungsgemäßen Polyurethan-Beschichtungen werden sogenannte Kettenverlängerer (d) eingesetzt. Solche Kettenverlängerer sind Di- oder Polyamine sowie Hydrazide, z.B. Hydrazin, 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4-Diaminodicyclohexylmethan, Dimethylethylendiamin, Hydrazin, Adipinsäuredihydrazid, 1,4-Bis(aminomethyl)cyclohexan, 4,4'-Diamino-3,3'-dimethyldicyclohexylmethan und andere (C₁-C₄)-Di- und Tetraalkyldicyclohexylmethane, z.B. 4,4'-Diamino-3,5-diethyl-3',5'-diisopropyldicyclohexylmethan.

Als Diamine oder Aminoalkohole kommen im Allgemeinen niedermolekulare Diamine oder Aminoalkohole in Betracht, die aktiven Wasserstoff mit gegenüber NCO-Gruppen unterschiedlicher Reaktivität enthalten, wie Verbindungen, die neben einer primären Aminogruppe auch sekundäre Aminogruppen oder neben einer Aminogruppe (primär oder sekundär) auch OH-Gruppen aufweisen. Beispiele hierfür sind primäre und sekundäre Amine, wie 3-Amino-1-Methylaminopropan, 3-Amino-1-Ethylaminopropan, 3-Amino-1-cyclohexylaminopropan, 3-Amino-1-Methylaminobutan, weiterhin Aminoalkohole, wie N-Aminoethylethanolamin, Ethanolamin, 3-Aminopropanol, Neopentanolamin und besonders bevorzugt Diethanolamin.

Der Bestandteil (d) der erfindungsgemäß zu verwendenden Beschichtungszusammensetzung kann bei deren Herstellung als Kettenverlängerer und/oder als Kettenterminierung eingesetzt werden.

Die Menge an Bestandteil (d) in der erfindungsgemäß zu verwendenden Beschichtungszusammensetzung beträgt vorzugsweise 0,05 bis 3,0 mol, besonders bevorzugt 0,1 bis 2,0 mol, insbesondere 0,2 bis 1,5 mol, jeweils bezogen auf den Bestandteil (a) der erfindungsgemäß zu verwendenden Beschichtungszusammensetzung.

### (e) Polyole

In einer weiteren Ausführungsform umfasst die Zusammensetzung der erfindungsgemäßen Beschichtung eines Polyurethanharnstoffes zusätzliche Einheiten, welche auf mindestens ein weiteres Polyol zurückgehen.

Die zum Aufbau der Polyurethanharnstoffe eingesetzten weiteren niedermolekularen Polyole (e) bewirken in der Regel eine Versteifung und oder eine Verzweigung der Polymerkette. Das Molekulargewicht beträgt vorzugsweise 62 bis 500 g/mol, besonders bevorzugt 62 bis 400 g/mol, insbesondere 62 bis 200 g/mol.

Geeignete Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z.B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiole wie z.B. α-Hydroxybutyl-ε-hydroxy-capronsäureester, ω-Hydroxyhexyl-γ-hydroxybuttersäure-ester, Adipinsäure-(β-hydroxyethyl)ester oder Terephthalsäure-bis(β-hydroxyethyl)-ester können verwendet werden.

Die Menge an Bestandteil (e) in der erfindungsgemäß zu verwendenden Beschichtungszusammensetzung beträgt vorzugsweise 0,1 bis 1,0 mol, besonders bevorzugt 0,2 bis 0,9 mol, insbesondere 0,2 bis 0,8 mol, jeweils bezogen auf den Bestandteil (a) der erfindungsgemäß zu verwendenden Beschichtungszusammensetzung.

### (f) Weitere amin- und/oder hydroxyenthaltende Bausteine (Aufbaukomponente)

Die Umsetzung der isocyanathaltigen Komponente (b) mit den hydroxy- oder aminfunktionellen Verbindungen (a), (c), (d) und gegebenenfalls (e) erfolgt üblicherweise unter Einhaltung eines leichten NCO-Überschusses gegenüber den reaktiven Hydroxy- oder Aminverbindungen. Durch die Dispergierung in Wasser werden Reste an Isocyanatgruppen zu Amingruppen hydrolisiert. Es kann aber im Einzelfall wichtig sein, den verbliebenen Rest an Isocyanatgruppen vor der Dispergierung des Polyurethans zu blockieren.

Die erfindungsgemäß vorgesehenen Polyurethanharnstoff-Beschichtungen können daher auch Aufbaukomponenten (f) enthalten, die sich jeweils an den Kettenenden befinden und diese abschließen. Diese Bausteine leiten sich zum einen von monofunktionellen, mit NCO-Gruppen reaktiven Verbindungen ab, wie Monoaminen, insbesondere mono-sekundären Aminen oder Monoalkoholen.

Genannt seien hier beispielsweise Ethanol, n-Butanol, Ethyienglykol-monobutylether, 2-Ethylhexanol, 1-Octanol, 1-Dodecanol, 1-Hexadecanol, Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin und geeignete substituierte Derivate davon.

Da die Bausteine (f) im Wesentlichen in den erfindungsgemäßen Beschichtungen dazu verwendet werden, den NCO-Überschuss zu vernichten, hängt die erforderliche Menge im Wesentlichen von der Menge des NCO-Überschusses ab und kann nicht allgemein spezifiziert werden.

### (g) Weitere Bestandteile

Die erfindungsgemäß vorgesehenen Polyurethanharnstoff-Beschichtungen können darüber hinaus weitere, für den angestrebten Zweck übliche Bestandteile wie Additive und Füllstoffe, enthalten. Ein Beispiel hierfür sind pharmakologische Wirkstoffe, Arzneimittel und Additive, welche die Freisetzung von pharmakologischen Wirkstoffen fördern ("drug-eluting-Additive").

Pharmakologische Wirkstoffe bzw. Arzneimittel, welche in den erfindungsgemäßen Beschichtungen auf den medizinischen Geräten verwendet werden können, sind im Allgemeinen beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel Zellzyklus-regulierende Mittel, genetische Mittel, Hormone, sowie deren Homologe, Derivate, Fragmente, pharmazeutische Salze und Kombinationen davon.

Spezifische Beispiele solcher Arzneimittel bzw. pharmakologischen Wirkstoffe schließen somit thromboresistente (nichtthrombogene) Mittel bzw. andere Mittel zur Unterdrückung einer akuten Thrombose, Stenose oder späten Re-Stenose der Arterien ein, beispielsweise Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw. Ein Wachstumsfaktor kann als ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle benutzt werden.

Das Arzneimittel bzw. der pharmakologische Wirkstoff kann auch aus einem Vasodilatator bestehen, um Vasospasmus entgegen zu wirken, zum Beispiel ein Antispasmusmittel wie Papaverin. Das Arzneimittel kann ein vasoaktives Mittel an sich sein, wie Calciumantagonisten, oder α- und β-adrenergische Agonisten oder Antagonisten. Zusätzlich kann das therapeutische Mittel ein biologisches Haftmittel wie Cyanoacrylat in medizinischer Qualität oder Fibrin, welche beispielsweise für das Ankleben einer Gewebeklappe an die Wand einer Koronararterie verwendet wird, sein.

Das therapeutische Mittel kann ferner ein antineoplastisches Mittel wie 5-Fluorouracil, vorzugsweise mit einem kontrollierenden freisetzenden Träger für das Mittel, sein (z.B. für die Anwendung eines fortdauernden kontrollierten freisetzenden antineoplastischen Mittels an einer Tumorstelle).

Das therapeutische Mittel kann ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. Ähnlich kann das therapeutische Mittel Steroide für den Zweck des Unterdrückens einer Entzündung in lokalisiertem Gewebe oder aus anderen Gründen enthalten.

Spezifische Beispiele geeigneter Arzneimittel umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze davon;
(b) antibiotischen Mitteln wie Penicilline, Cephalosprine, Vacomycine, Aminoglycoside, Quinolone, Polymxine, Erythromycine; Tertracycline, Chloramphenicole, Clindamycine, Lincomycine, Sulfonamide, deren Homologe, Analoge, Derivate, pharmazeutische Salze und Mischungen davon;
(c) Paclitaxel, Docetaxel, Immunsuppressiva wie Sirolimus oder Everolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen davon; und
(d) antivrale Mittel wie Amantadin, Rimantadin, Rabavirin, Idoxuridin, Vidarabin, Trifluridin, Acyclovir, Ganciclocir, Zidovudin, Phosphonoformate, Interferone, deren Homologe, Analoge, Fragmente, Derivate, pharmazeutische Salze und Mischungen davon; und
(e) entzündungshemmende Mittel wie beispielsweise Ibuprofen, Dexamethason oder Methylprednisolon.

In einer bevorzugten Ausführungsform umfasst die erfindungsgemäß vorgesehene Beschichtungszusammensetzung einen Polyurethanharnstoff, welcher aufgebaut wird aus
a) mindestens einem Polycarbonatpolyol;
b) mindestens einem Polyisocyanat;
c) mindestens einem monofunktionellen gemischten Polyoxyalkylenether aus Polyethylenoxid und Polypropylenoxid, und
d) mindestens einem Diamin oder einem Aminoalkohol.

In einer weiteren bevorzugten Ausführungsform umfasst die erfindungsgemäße Beschichtungszusammensetzung einen Polyurethanharnstoff, welcher aufgebaut wird aus
a) mindestens einem Polycarbonatpolyol;
b) mindestens einem Polyisocyanat;
c) mindestens einem monofunktionellen gemischten Polyoxyalkylenether aus Polyethylenoxid und Polypropylenoxid;
d) mindestens einem Diamin oder einem Aminoalkohol; und
e) mindestens einem Polyol.

In einer weiteren Ausführungsform der vorliegenden Erfindung umfasst die erfindungsgemäß vorgesehene Beschichtungszusammensetzung einen Polyurethanharnstoff, welcher aufgebaut wird aus
a) mindestens einem Polycarbonatpolyol;
b) mindestens einem Polyisocyanat;
c) mindestens einem monofunktionellen gemischten Polyoxyalkylenether aus Polyethylenoxid und Polypropylenoxid;
d) mindestens einem Diamin oder einem Aminoalkohol;
e) mindestens einem Polyol; und
f) mindestens einem amin- oder hydroxylhaltiges Monomer, welches sich an den Polymerkettenenden befindet.

Erfindungsgemäß werden zur Beschichtung der medizinischen Geräte besonders bevorzugt Polyurethanharnstoffe eingesetzt, die aufgebaut werden aus
a) mindestens einem Polycarbonatpolyol mit einem mittleren Molgewicht zwischen 400 g/mol und 6000 g/mol und einer Hydroxylfunktionalität von 1,7 bis 2,3 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polycarbonatpolyols von 1,0 bis 4,0 mol;
c) mindestens einem monofunktionellen gemischten Polyoxyalkylenether aus Polyethylenoxid und Polypropylenoxid oder einer Mischung solcher Polyether mit einem mittleren Molgewicht zwischen 500 g/mol und 5000 g/mol in einer Menge pro Mol des Polycarbonatpolyols von 0,01 bis 0,5 mol;
d) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,05 bis 3,0 mol;
e) gegebenenfalls einem oder mehreren kurzkettigen aliphatischen Polyolen mit einem Molgewicht zwischen 62 g/mol und 500 g/mol in einer Menge pro Mol des Polycarbonatpolyols von 0,1 bis 1,0 mol; und
f) gegebenenfalls amin- oder OH haltige Bausteine, die sich an den Polymerkettenenden befinden und diese abschließen.

Erfindungsgemäß weiter bevorzugt werden zur Beschichtung von medizinischen Geräten Polyurethanharnstoffe eingesetzt, die aufgebaut werden aus
a) mindestens einem Polycarbonatpolyol mit einem mittleren Molgewicht zwischen 500 g/mol und 5000 g/mol und einer Hydroxylfunktionalität von 1,8 bis 2,2 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polycarbonatpolyols von 1,2 bis 3,8 mol;
c) mindestens einem monofunktionellen gemischten Polyoxyalkylenether aus Polyethylenoxid und Polypropylenoxid oder einer Mischung solcher Polyether mit einem mittleren Molgewicht zwischen 1000 g/mol und 4000 g/mol in einer Menge pro Mol des Polycarbonatpolyols von 0,02 bis 0,4 mol;
d) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,1 bis 2,0 mol;
e) gegebenenfalls einem oder mehreren kurzkettigen aliphatischen Polyolen mit einem Molgewicht zwischen 62 g/mol und 400 g/mol in einer Menge pro Mol des Polycarbonatpolyols von 0,2 bis 0,9 mol; und
f) gegebenenfalls amin- oder OH haltige Bausteine, die sich an den Polymerkettenenden befinden und diese abschließen.

Erfindungsgemäß noch weiter bevorzugt werden zur Beschichtung von Kathetermaterialien Polyurethanharnstoffe eingesetzt, die aufgebaut werden aus
a) mindestens einem Polycarbonatpolyol mit einem mittleren Molgewicht zwischen 600 g/mol und 3000 g/mol und einer Hydroxylfunktionalität von 1,9 bis 2,1 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polycarbonatpolyols von 1,5 bis 3,5 mol;
c) mindestens einem monofunktionellen gemischten Polyoxyalkylenether aus Polyethylenoxid und Polypropylenoxid oder einer Mischung solcher Polyether mit einem mittleren Molgewicht zwischen 1000 g/mol und 3000 g/mol in einer Menge pro Mol des Polycarbonatpolyols von 0,04 bis 0,3 mol;
d) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,2 bis 1,5 mol;
e) gegebenenfalls einem oder mehreren kurzkettigen aliphatischen Polyolen mit einem Molgewicht zwischen 62 g/mol und 200 g/mol in einer Menge pro Mol des Polycarbonatpolyols von 0,2 bis 0,8 mol; und
f) gegebenenfalls amin- oder OH haltige Bausteine, die sich an den Polymerkettenenden befinden und diese abschließen.

Die Beschichtungszusammensetzung wird auf einem medizinischen Gerät aufgebracht.

### Medizinisches Gerät

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Kathetern, zum Beispiel urologische Kathetern wie Blasenkathetern oder Harnleiterkathetern; zentralvenöse Katheter; venöse Kathetern oder Einlass- bzw. Auslass-Kathetern; Dilationsballons; Kathetern für die Angioplastie und die Biopsie; Kathetern, die für das Einbringen eines Stents, eines Propf- oder eines Kavafilters verwendet werden; Ballonkathetern oder andere dehnbare medizinische Geräte; Endoskope; Laryngoskope; Trachealgeräte wie Endotrachealschläuche, Atemgeräte und andere Trachealabsauggeräte; bronchoalveolare Spülungskathetern; Kathetern, die bei der Koronarangioplastie verwendet werden; Führungsstäbe, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Darüber hinaus können die erfindungsgemäßen Beschichtungslösungen zur Herstellung von Schutzbeschichtungen, zum Beispiel für Handschuhe, Stents und andere Implantate; extrakorporale (außerkörperliche) Blutschläuche (Blutführungsrohre); Membrane, zum Beispiel für die Dialyse; Blutfilter; Geräte für die Kreislaufunterstützung; Verbandsmaterial für die Wundpflege; Harnbeutel und Stomabeutel verwendet werden. Eingeschlossen sind auch Implantate, die ein medizinisch wirksames Mittel enthalten, wie medizinisch wirksame Mitteln für Stents oder für Ballonoberflächen oder für Kontrazeptiva.

Üblicherweise wird das medizinisches Gerät aus Kathetern, Endoskopen, Laryngoskopen, Endotrachealschläuchen, Ernährungsschläuchen, Führungsstäben, Stents, und andere Implantate gebildet.

Als Substrat der zu beschichtenden Oberfläche kommen viele Materialien in Frage, wie Metalle, Textilien, Keramiken oder Kunststoffe, wobei die Verwendung von Kunststoffen für die Herstellung von medizinischen Geräten bevorzugt ist.

Erfindungsgemäß wurde gefunden, dass man medizinische Geräte mit sehr hydrophilen und damit gleitfähigen, blutverträglichen Oberflächen erzeugen kann, indem man zur Beschichtung der medizinischen Geräte wässrige, nichtionisch stabilisierte Polyurethandispersionen der oben beschriebenen Art verwendet. Die oben beschriebenen Beschichtungszusammensetzungen werden vorzugsweise als wässrige Dispersionen erhalten und auf die Oberfläche der medizinischen Geräte appliziert.

### Herstellung der Beschichtungslösung

Die oben näher beschriebenen Bestandteile der Beschichtungen werden im Allgemeinen so umgesetzt, dass zunächst ein harnstoffgruppenfreies, isocyanatfunktionelles Prepolymer durch Umsetzung der Bestandteile (a), (b), (c) und gegebenenfalls (e) hergestellt wird, wobei das Stoffmengenverhältnis von Isocyanatgruppen zu mit Isocyanat reaktiven Gruppen des Polycarbonatpolyols vorzugsweise 0,8 bis 4,0, besonders bevorzugt 0,9 bis 3,8 insbesondere 1,0 bis 3,5 beträgt.

In einer alternativen Ausführungsform kann auch erst der Bestandteil (a) separat mit dem Isocyanat (b) umgesetzt werden. Danach kann dann die Zugabe der Bestandteile (c) und gegebenenfalls (e) und deren Umsetzung erfolgen. Anschließend werden im Allgemeinen die verbliebenen Isocyanat-Gruppen vor, während oder nach dem Dispergieren in Wasser aminofunktionell kettenverlängert oder terminiert, wobei das Äquivalentverhältnis von isocyanatreaktiven Gruppen der zur Kettenverlängerung eingesetzten Verbindungen zu freien Isocyanat-Gruppen des Prepolymers vorzugsweise zwischen 40 bis 150 %, besonders bevorzugt zwischen 50 bis 120 %, insbesondere zwischen 60 bis 120 %, liegt (Bestandteil (d)).

Die erfindungsgemäßen Polyurethandispersionen werden dabei vorzugsweise nach dem sogenannten Aceton-Verfahren hergestellt. Für die Herstellung der Polyurethandispersion nach diesem Aceton-Verfahren werden üblicherweise die Bestandteile (a), (c) und (e), die keine primären oder sekundären Aminogruppen aufweisen dürfen und die Polyisocyanatkomponente (b) zur Herstellung eines isocyanatfunktionellen Polyurethan-Prepolymers ganz oder teilweise vorgelegt und gegebenenfalls mit einem mit Wasser mischbaren, aber gegenüber Isocyanatgruppen inerten Lösungsmittel verdünnt und auf Temperaturen im Bereich von 50 bis 120 °C aufgeheizt. Zur Beschleunigung der Isocyanatadditionsreaktion können die in der Polyurethan-Chemie bekannten Katalysatoren eingesetzt werden, beispielsweise Dibutylzinndilaurat. Bevorzugt ist die Synthese ohne Katalysator.

Geeignete Lösungsmittel sind die üblichen aliphatischen, ketofunktionellen Lösemittel wie z.B. Aceton, Butanon, die nicht nur zu Beginn der Herstellung, sondern gegebenenfalls in Teilen auch später zugegeben werden können. Bevorzugt sind Aceton und Butanon. Andere Lösemittel wie z.B. Xylol, Toluol, Cyclohexan, Butylacetat, Methoxypropylacetat, Lösemittel mit Ether- oder Estereinheiten können ebenfalls eingesetzt und ganz oder teilweise abdestilliert werden oder vollständig in der Dispersion verbleiben.

Anschließend werden die gegebenenfalls zu Beginn der Reaktion noch nicht zugegebenen Bestandteile von (c) und (e) zudosiert.

In einer bevorzugten Weise wird das Prepolymer ohne Lösungsmittelzusatz hergestellt und erst für die Kettenverlängerung mit einem geeigneten Lösungsmittel, vorzugsweise Aceton, verdünnt.

Bei der Herstellung des Polyurethan-Prepolymeren beträgt das Stoffmengenverhältnis von Isocyanatgruppen zu mit Isocyanat reaktiven Gruppen vorzugsweise 0,8 bis 4,0, besonders bevorzugt 0,9 bis 3,8, insbesondere 1,0 bis 3,5.

Die Umsetzung zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss wird in einem weiteren Verfahrensschritt, falls noch nicht oder nur teilweise geschehen, das erhaltene Prepolymer mit Hilfe von aliphatischen Ketonen wie Aceton oder Butanon gelöst.

Anschließend werden mögliche NH₂-, NH-funktionelle und/oder OH-funktionelle Komponenten mit den noch verbliebenen Isocyanatgruppen umgesetzt. Diese Kettenvertängerung/-terminierung kann dabei entweder in Lösungsmittel vor dem Dispergieren, während des Dispergierens oder in Wasser nach dem Dispergieren durchgeführt werden. Bevorzugt wird die Kettenverlängerung vor der Dispergierung in Wasser durchgeführt.

Werden zur Kettenverlängerung Verbindungen entsprechend der Definition von (d) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung eingesetzten Verbindungen zu freien NCO-Gruppen des Prepolymers liegt vorzugsweise zwischen 40 bis 150 %, besonders bevorzugt zwischen 50 bis 120 %, insbesondere zwischen 60 bis 120 %.

Die aminischen Komponenten (d) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mitverwendet werden, so beträgt der Verdünnungsmittelgehalt bevorzugt 70 bis 95 Gew.-%.

Die Herstellung der Polyurethandispersion aus den Prepolymeren erfolgt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den Prepolymerlösungen gerührt. Bevorzugt wird das Wasser in das gelöste Prepolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Feststoffgehalt der Polyurethandispersion nach der Synthese liegt zwischen 20 bis 70 Gew.-%, bevorzugt 20 bis 65 Gew.-%. Für Beschichtungsversuche können diese Dispersionen beliebig mit Wasser verdünnt werden, um die Dicke der Beschichtung variabel einstellen zu können. Alle Konzentrationen von 1 bis 60 Gew.-% sind möglich, bevorzugt sind Konzentrationen im Bereich 1 bis 40 Gew.-%.

Dabei können beliebige Schichtdicken erreicht werden, wie beispielsweise einige 100 nm bis hinauf zu einigen 100 µm, wobei im Rahmen der vorliegenden Erfindung auch höhere und geringere Dicken möglich sind.

Die Polyurethanmaterialien für die Beschichtung der medizinischen Geräte können durch Verdünnung der erfindungsgemäßen wässrigen Dispersionen mit Wasser auf jeden gewünschten Wert verdünnt werden. Darüber hinaus können Verdicker zugesetzt werden, um die Viskosität der Polyurethandispersionen gegebenenfalls erhöhen zu können. Weitere Zusätze wie beispielsweise Antioxidantien, Puffermaterialien zur Einstellung des pH-Wertes oder Pigmente sind ebenfalls möglich. Darüber hinaus können gegebenenfalls noch weitere Zusätze wie Griffhilfsmittel, Farbstoffe, Mattierungsmittel, UV-Stabilisatoren, Lichtstabilisatoren, weitere Hydrophilierungsmittel, Hydrophobierungsmittel und/oder Verlaufshilfsmittel verwendet werden.

### Herstellung der Beschichtungen

Im Rahmen der vorliegenden Erfindung ist es insbesondere bevorzugt, dass die Beschichtungen der medizinischen Geräte ausgehend von Dispersionen der oben näher beschriebenen Beschichtungszusammensetzung hergestellt werden. Die Dispersion wird vorzugsweise wie oben beschrieben erhalten.

Erfindungsgemäß hat sich herausgestellt, dass sich die resultierenden Beschichtungen auf medizinischen Geräten unterscheiden, je nachdem, ob die Beschichtung ausgehend von einer Dispersion oder einer Lösung hergestellt wird.

Dabei weisen die erfindungsgemäßen Beschichtungen auf medizinischen Geräten dann Vorteile auf, wenn sie ausgehend von Dispersionen der oben beschriebenen Beschichtungszusammensetzungen erhalten werden, da Dispersionen der erfindungsgemäßen Beschichtungssystemen zu Beschichtungen auf den medizinischen Geräten führen, die keine organischen Lösemittelreste aufweisen, das heißt toxisch im Allgemeinen unbedenklich sind, und gleichzeitig zu einer ausgeprägteren Hydrophilie führen, was sich beispielsweise an einem geringen Kontaktwinkel ausmachen lässt. Hierzu wird auf die weiter untenstehend erläuterten Versuche und Vergleichsversuche verwiesen.

In einer weiteren Ausgestaltung betrifft die vorliegende Erfindung daher ein medizinisches Gerät mit mindestens einer hydrophilen Beschichtung, enthaltend mindestens einen Polyurethanharnstoff, wobei die Beschichtung ausgehend von einer Dispersion des Polyurethanharnstoff hergestellt wird. Der Polyurethanharnstoff ist vorzugsweise der oben beschriebene erfindungsgemäße Polyurethanhamstoff.

Die erfindungsgemäßen medizinischen Geräte können dabei mittels verschiedener Verfahren mit den hydrophilen Polyurethandispersionen beschichtet werden. Geeignete Beschichtungstechniken sind hierfür beispielsweise Rakeln, Drucken, Transferbeschichten, Sprühen, Spincoating oder Tauchen.

Die wässrigen Polyurethandispersionen, welche als Ausgangsstoff für die Herstellung der Beschichtungen verwendet werden, können nach beliebigen Verfahren hergestellt werden, wobei jedoch das zuvor beschriebene Acetonverfahren bevorzugt ist.

Beschichtet werden können dabei vielerlei Substrate wie Metalle, Textilien, Keramiken und Kunststoffe. Bevorzugt ist die Beschichtung von medizinischen Geräten, die aus Metallen oder aus Kunststoff gefertigt sind. Als Metalle können beispielsweise genannt werden: Medizinischer Edelstahl oder Nickel-Titan-Legierungen. Es sind viele Polymermaterialien denkbar, aus denen das medizinische Geräte aufgebaut sein kann, beispielsweise Polyamid; Polystyrol; Polycarbonat; Polyether; Polyester; Polyvinylacetat; natürliche und synthetische Kautschuke; Blockcopolymere aus Styrol und ungesättigten Verbindungen wie Ethylen, Butylen und Isopren; Polyethyten oder Copolymeren aus Polyethyten und Polypropyten; Silikon; Polyvinylchlorid (PVC) und Polyurethanen. Zur besseren Haftung des hydrophilen Polyurethane auf dem medizinischen Gerät können als Untergrund vor dem Auftragen dieser hydrophilen Beschichtungsmaterialien noch weitere geeignete Beschichtungen aufgetragen werden.

Zusätzlich zu den hydrophilen Eigenschaften der Verbesserung der Gleitfähigkeit zeichnen sich die erfindungsgemäß vorgesehenen Beschichtungszusammensetzungen auch durch eine hohe Blutverträglichkeit aus. Hierdurch ist auch ein Arbeiten mit diesen Beschichtungen besonders im Blutkontakt vorteilhaft. Die Materialien zeigen im Vergleich zu Polymeren des Standes der Technik eine reduzierte Gerinnungsneigung im Blutkontakt.

Die Vorteile der erfindungsgemäßen Katheter mit den hydrophilen Polyurethanbeschichtungen werden durch Vergleichsversuche in den folgenden Beispielen dargelegt.

### Beispiele

Die Ermittlung des NCO-Gehaltes der in den Beispielen und Vergleichsbeispielen beschriebenen Harze erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvem Instruments.

Die in % angegebenen Mengenangaben verstehen sich, wenn nicht anders vermerkt, als Gew.-% und beziehen sich auf die erhaltene wässrige Dispersion.

### Verwendete Substanzen und Abkürzungen:

- Desmophen C2200:: Polycarbonatpolyol, OH-Zahl 56 mg KOH/g, zahlenmittleres Molekulargewicht 2000 g/mol (Bayer, MaterialScience AG, Leverkusen, DE)
- Desmophen C1200:: Polycarbonatpolyol, OH-Zahl 56 mg KOH/g, zahlenmittleres Molekulargewicht 2000 g/mol (Bayer MaterialScience AG, Leverkusen, DE)
- Desmophen XP 2613: Polycarbonatpolyol, OH-Zahl 56 mg KOH/g, zahlenmittleres Molekulargewicht 2000 g/mol (Bayer MaterialScience AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- Polyether LB 25:: (monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE)

### Beispiel 1:

Dieses Beispiel beschreibt die Herstellung einer erfindungsgemäßen Polyurethanharnstoff-Dispersion.

277,2 g Desmophen C 2200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 3 h 40 min war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 15 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,5 % und einer mittleren Teilchengröße von 164 nm erhalten.

### Beispiel 2:

Dieses Beispiel beschreibt die Herstellung einer erfindungsgemäßen Polyurethanharnstoff-Dispersion.

269,8 g Desmophen C 2200, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 100 °C. Nach 21,5 h war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,3 % und einer mittleren Teilchengröße von 109 nm erhalten.

### Beispiel 3:

Dieses Beispiel beschreibt die Herstellung einer erfindungsgemäßen Polyurethanharnstoff-Dispersion.

277,2 g Desmophen C 1200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 2,5 h war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 40,4 % und einer mittleren Teilchengröße von 146 nm erhalten.

### Beispiel 4:

Dieses Beispiel beschreibt die Herstellung einer erfindungsgemäßen Polyurethanharnstoff-Dispersion.

282,1 g Desmophen C 2200, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 21,5 h war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,7 % und einer mittleren Teilchengröße von 207 nm erhalten.

### Beispiel 5:

Dieses Beispiel beschreibt die Herstellung einer erfindungsgemäßen Polyurethanharnstoff-Dispersion.

269,8 g Desmophen XP 2613, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 70 min war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,2 % und einer mittleren Teilchengröße von 112 nm erhalten.

### Beispiel 6:

Dieses Beispiel beschreibt die Herstellung einer erfindungsgemäßen Polyurethanharnstoff-Dispersion.

249,4 g Desmophen C 2200, 33,1 g Polyether LB 25, 1,9 g Trimethylolpropan und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 4 h 20 min war der theoretische NCO-Wert erreicht. Das fertige Prepolymer wurde bei 50 °C in 720 g Aceton gelöst und anschließend bei 40 °C eine Lösung aus 3,3 g Ethylendiamin in 16 g Wasser innerhalb von 10 min zudosiert. Die Nachrührzeit betrug 15 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 38,9 % und einer mittleren Teilchengröße von 144 nm erhalten.

### Beispiel 7:

282,1 g Desmophen XP 2613, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 70 min war der theoretische NCO-Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile

Polyurethandispersion mit einem Festkörpergehalt von 38,3 % und einer mittleren Teilchengröße von 215 nm erhalten.

### Beispiel 8:

Dieses Beispiel beschreibt die Herstellung einer Polyurethanharnstoff-Dispersion als Vergleichsprodukt zu dem erfindungsgemäßen Beispiel 1. Das Desmophen C2200 wird durch PolyTHF 2000 ausgetauscht.

277,2 g PolyTHF 2000, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 18 h war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 40,7 % und einer mittleren Teilchengröße von 166 nm erhalten.

### Beispiel 9:

Dieses Beispiel beschreibt die Herstellung einer Polyurethanharnstoff-Dispersion als Vergleichsprodukt zu dem erfindungsgemäßen Beispiel 2. Das Desmophen C2200 wird durch das PolyTHF 2000 ausgetauscht.

269,8 g PolyTHF 2000, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 100 °C. Nach 17,5 h war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,6 % und einer mittleren Teilchengröße von 107 nm erhalten.

### Beispiel 10:

Dieses Beispiel beschreibt die Herstellung einer Polyurethanharnstoff-Dispersion als Vergleichsprodukt zu dem erfindungsgemäßen Beispiel 4. Das Desmophen C2200 wird durch das PolyTHF 2000 ausgetauscht.
282,1 g PolyTHF 2000, 22,0 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 21,5 h war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 37,5 % und einer mittleren Teilchengröße von 195 nm erhalten.

### Beispiel 11: Herstellung der Beschichtungen und Messung des statischen Kontaktwinkels

Die Beschichtungen zur Messung des statischen Kontaktwinkels wurden auf Glasobjektträger der Größe 25x75 mm mit Hilfe eines Spincoaters (RC5 Gyrset 5, Karl Süss, Garching, Deutschland) hergestellt. Ein Objektträger wurde hierzu auf dem Probenteller des Spincoaters eingespannt und mit ca. 2,5 - 3 g wässriger unverdünnter Polyurethandispersion homogen bedeckt. Durch Rotation des Probentellers für 20 sec bei 1300 Umdrehungen pro Minute erhielt man eine homogene Beschichtung, die 15 min bei 100 °C und danach 24 h bei 50 °C getrocknet wurde. Die erhaltenen beschichteten Objektträger wurden direkt einer Kontaktwinkelmessung unterzogen.

Von den erhaltenen Beschichtungen auf den Objektträgern wird eine statische Kontaktwinkelmessung durchgeführt. Mittels des Video- Randwinkelmessgerätes OCA20 der Firma Dataphysics mit rechnergesteuerten Spritzen wird auf das Muster 10 Tropfen Milliporewasser aufgesetzt und deren statischer Benetzungsrandwinkel gemessen. Zuvor wird mittels eines Antistatikföns die statische Aufladung (falls vorhanden) auf der Probenoberfläche entfernt.

**Tabelle 1: Statische Kontaktwinkelmessungen**

| PU-FILM | KONTAKTWINKEL [°] |
|---|---|
| Beispiel 1 | <10 |
| Beispiel 2 | 11 |
| Beispiel 3 | 14 |
| Beispiel 4 | 20 |
| Beispiel 5 | 14 |
| Beispiel 6 | 26 |
| Beispiel 7 | 41 |
| Vergleichsbeispiel 8 | 66 |
| Vergleichsbeispiel 9 | 62 |
| Vergleichsbeispiel 10 | 77 |

Wie Tabelle 1 zeigt, ergeben die polycarbonathaltigen Beschichtungen der erfindungsgemäßen Beispiele 1 bis 7 äußerst hydrophile Beschichtungen mit statischen Kontaktwinkeln s 45 °. Die Beschichtungen der Beispiele 1 bis 6 ergeben außerordentlich hydrophile Beschichtungen mit statischen Kontaktwinkeln < 30 °. Dagegen sind die PolyTHF-haltigen Beschichtungen aus den Vergleichsbeispielen 7 bis 10 wesentlich unpolarer, obwohl die Zusammensetzung dieser Beschichtungen ansonsten mit denen der Beispiele 1, 2 und 4 identisch sind.

Darüber hinaus zeigen Daten, die in "Evaluation of a poly(vinylpyrollidone)-coated biomaterial for urological use"; M.M. Tanney, S.P. Gorman, Biomaterials 23 (2002), 4601 - 4608, offenbart sind, dass die Kontaktwinkel von Polyurethan bei ca. 97° und von mit PVP beschichtetem Polyurethan bei ca. 50° liegt.

### Beispiel 12: Messung von Gerinnungsparametern

Aus der Polyurethandispersion des Beispiels 1 wurde durch Spincoaten auf Glas ein Film für Studien zum Blutkontakt hergestellt. Die Probenoberfläche wurde in eine autoklavierten Inkubationskammer eingelegt und mit 1,95 ml Blut inkubiert. Der genaue Versuchsaufbau ist in U. Streller et al. J. Biomed. Mater. Res B, 2003, 66B, 379-390 beschrieben.

Das für den Versuch benötigte venöse Blut wurde über eine 19 G Kanüle einem männlichen Spender entnommen, der mindestens 10 Tage keine Medikamente eingenommen hat. Die Gerinnung wurde durch Zugabe von Heparin (2 iU/ml) verhindert. Das so vorbereitete Blut wurde dann in die mit der Polyurethanoberfläche bestückte, auf 37 °C vortemperierte Inkubationskammer gefüllt und 2 h bei permanenter Rotation der Kammer bei 37 °C inkubiert. Als Vergleichsmaterialien wurden Glas und Polytetrafluorethylen (PTFE) verwendet. Glas ist für die Blutgerinnung eine stark aktivierende Oberfläche, während PTFE ein Polymer ist, welches für viele Anwendungen ein akzeptables Material ist (siehe U. Streller et al. J. Biomed. Mater. Res B, 2003, 66B, 379-390).

Nach erfolgter Inkubation wurden drei Parameter gemessen:
Thrombin-Antithrombin-Komplex (Enzygnost TAT micro, Dade Behring GmbH, Marburg, Deutschland)
Plättchenfaktor 4 (ELISA PF 4 Komplettkit der Haemochrom Diagnostica GmbH, Essen, Deutschland)

Die Thrombocytenreduktion wurde in EDTA-antikoaguliertem Blut mittels eines automatischen Zellzählsystems (AcTdiff der Firma Coulter, Krefeld, Deutschland) gemessen.

**Tabelle 2: Thrombin-Antithrombin-Komplex**

| Oberfläche | Thrombin-Antithrombin-Komplex (µg/mL) |
|---|---|
| Polyurethan des Beispiels 1 | 27,7 |
| PTFE | 33,4 |

**Tabelle 3: Plättchenfaktor 4**

| Oberfläche | Thrombin-Antithromin-Komplex (IU/mL) |
|---|---|
| Polyurethan des Beispiels 1 | 29,7 |
| Glas | 377,2 |
| PTFE | 59,2 |

**Tabelle 4: Thrombozytenreduktion im Blut**

| Oberfläche | Thromborytenzahl (% Reduktion) |
|---|---|
| Polyurethan des Beispiels 1 | -0,3 |
| Glas | 17,9 |
| PTFE | 5,7 |

Alle drei gemessenen Blutparameter zeigen, dass das hydrophile Polyurethan des Beispiels 1 die Gerinnung nur in sehr maßvoller Weise aktiviert. Der Thrombin-Antithrombin-Komplex als Maß für die Aktivierung der intrinsischen Gerinnungskaskade zeigt, dass das Polyurethan selbst im Vergleich zu dem als sehr gut blutverträglich angesehenen PTFE geringere Werte erzeugt und dadurch eine noch geringere Aktivierung hervorruft.

Plättchenfaktor 4 ist ein Marker für die Aktivierung der Thrombozyten. Auch dieser zelluläre Teil der Gerinnung wird nur in geringem Maß von dem hydrophilen Polyurethan aktiviert. Das gut blutverträgliche PTFE ruft eine höhere Aktivierung hervor. Auch die Reduktion der Thrombozyten ist für Glass und auch PTFE deutlich, was bedeutet, dass sich ein Teil der Thrombozyten an diesen Oberflächen anlagern. Demgegenüber ist beim hydrophilen Polyurethan des Beispiels 1 praktisch kein Abfall zu erkennen.

### Beispiel 13:

Dieses Beispiel beschreibt die Synthese einer wässrigen Dispersion mit terminalen Polyethlyenoxidbausteinen als Vergleichs material zu den erfindungsgemäßen Beispielen, in welchen ein Polyurethan verwendet wird, welches durch ein Copolymer aus Polyethylenoxid und Polypropylenoxid terminiert ist. Der im Sinne der vorliegenden Erfindung verwendete Polyether LB 25 wird in diesem Beispiel durch gleiche molaren Mengen eines vergleichbaren reinen Polyethylenoxidethers ausgetauscht.

277,2 g Desmophen C 2200, 29,4 g Polyethylenglykol-2000-monomethylether (Quelle: Fluka, CAS Nr. 9004-74-4) und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 35 min war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 40,0 % und einer mittleren Teilchengröße von 130 nm erhalten.

Wie unter Beispiel 11 beschrieben, wurde durch Spincoating eine Beschichtung auf Glas hergestellt und der statische Kontaktwinkel dieser Beschichtung ermittelt. Man erhielt einen statischen Kontaktwinkel von 45 °. Der Vergleich dieses Wertes mit dem Wert für die Beschichtung des Beispiels 1 (< 10 °, siehe Tabelle 1 im Beispiel 11) zeigt, dass die Verwendung des gemischten Polyethylenoxid-Polypropylenoxid-Monols LB 25 im Vergleich zum reinen Polyethylenoxidmonool deutlich niedrigere Kontaktwinkel und somit hydrophilere Beschichtungen ermöglicht.

### Beispiel 14:

Dieses Beispiel beschreibt die Synthese des Polyurethanharnstoffpolymers des erfindungsgemäßen Beispiels 1 als Vergleichsbeispiel in organischer Lösung.

Zu einer Mischung aus 277,2 g Desmophen C 2200, 33,1 g LB 25, 6,7 g Neopentylglykol werden bei 60 °C 71,3 g 4,4'-Bis(isocyanatocyclohexyl)methan (H₁₂MDI) und 11,9 g Isophorondiisocyanat zugegeben. Man erwärmte die Mischung auf 110 °C und setzte bis zu einem konstanten NCO-Gehalt von 2,4 um. Man ließ abkühlen und verdünnte mit 475 g Toluol und 320 g iso-Propanol. Bei Raumtemperatur wurden eine Lösung von 4,8 g Ethylendiamin in 150 g 1-Methoxypropanol-2 innerhalb zugegeben. Nach vollständiger Zugabe wurde 2 h nachgerührt. Man erhielt 1350 g einer 30,2%igen Polyurethanharnstofflösung in Toluol/iso-Propanol/1-Methoxypropanol-2 mit einer Viskosität von 607 mPas bei 23 °C.

Wie unter Beispiel 11 beschrieben, wurde durch Spincoating eine Beschichtung auf Glas hergestellt und der statische Kontaktwinkel dieser Beschichtung ermittelt. Man bestimmte einen statischen Kontaktwinkel von 27 °. Der Vergleich dieses Wertes mit dem Wert für die Beschichtung des Beispiels 1 (< 10 °, siehe Tabelle 1 im Beispiel 11), einer strukturell gleichen Beschichtung, aber in Wasser dispergiert, zeigt, dass die Beschichtungen aus wässriger Dispersion im Vergleich zu Beschichtung, welche ausgehend von entsprechenden Lösungen erhalten werden, hydrophilere Beschichtungen ergeben.

## Patentansprüche

1. Medizinisches Gerät mit mindestens einer Beschichtung, enthaltend mindestens einen Polyurethanharnstoff,
**dadurch gekennzeichnet, dass**
der Polyurethanharnstoff mit einer Copolymereinheit aus Polyethylenoxid und Polypropylenoxid terminiert ist.

2. Medizinisches Gerät nach Anspruch 1, **dadurch gekennzeichnet, dass** der Polyurethanharnstoff Einheiten aufweist, welche auf mindestens ein Hydroxylgruppen enthaltendes Polycarbonat zurückgehen.

3. Medizinisches Gerät nach Anspruch 1 oder 2, **dadurch gekennzeichnet, dass** der Polyurethanharnstoff Einheiten aufweist, welche auf mindestens ein aliphatisches, cycloaliphatisches oder aromatisches Isocyanat zurückgehen.

4. Medizinisches Gerät nach einem der Ansprüche 1 bis 3, **dadurch gekennzeichnet, dass** der Polyurethanharnstoff Einheiten aufweist, die auf mindestens ein Diamin oder Aminalkohol zurückgehen.

5. Medizinisches Gerät nach einem der Ansprüche 1 bis 4, **dadurch gekennzeichnet, dass** die Beschichtung einen Polyurethanharnstoff aufweist, welcher aufgebaut ist aus
a) mindestens einem Polycarbonatpolyol mit einem mittleren Molgewicht zwischen 400 g/mol und 6000 g/mol und einer Hydroxylfunktionalität von 1,7 bis 2,3 oder aus Mischungen solcher Polycarbonatpolyole;
b) mindestens einem aliphatischen, cycloaliphatischen oder aromatischen Polyisocyanat oder Mischungen solcher Polyisocyanate in einer Menge pro Mol des Polycarbonatpolyols von 1,0 bis 4,0 mol;
c) mindestens einem monofunktionellen gemischten Polyoxyalkylenether aus Polyethylenoxid und Polypropylenoxid oder einer Mischung solcher Polyether mit einem mittleren Molgewicht zwischen 500 g/mol und 5000 g/mol in einer Menge pro Mol des Polycarbonatpolyols von 0,01 bis 0,5 mol;
d) mindestens einem aliphatischen oder cycloaliphatischen Diamin oder mindestens ein Aminoalkohol als so genannte Kettenverlängerer oder Mischungen solcher Verbindungen in einer Menge pro Mol des Polycarbonatpolyols von 0,05 bis 3,0 mol;
e) gegebenenfalls einem oder mehreren kurzkettigen aliphatischen Polyolen mit einem Molgewicht zwischen 62 g/mol und 500 g/mol in einer Menge pro Mol des Polycarbonatpolyols von 0,1 bis 1,0 mol; und
f) gegebenenfalls amin- oder OH haltige Bausteine, die sich an den Polymerkettenenden befinden und diese abschließen.

6. Verfahren zur Herstellung eines medizinischen Gerätes mit mindestens einer Beschichtung, wobei die Beschichtung erhältlich ist ausgehend von einer Dispersion, enthaltend mindestens einen Polyurethanharnstoff gemäß der Definition in einem der Ansprüche 1 bis 5.

7. Verfahren nach Anspruch 6, **dadurch gekennzeichnet, dass** die Beschichtung ausgehend von einer Dispersion des Polyurethanharnstoffes durch Rakeln, Drucken, Transferbeschichten, Sprühen, Spincoating öder Tauchen auf dem medizinischen Gerät aufgebracht wird.

8. Medizinisches Gerät, erhältlich nach Anspruch 6 oder 7.

9. Medizinisches Gerät nach einem der Ansprüche 1 bis 5 oder 8 in der Form von Kontaktlinsen; Kanülen; Kathetern, insbesondere urologischen Kathetern wie Blasenkathetern oder Harnleiterkathetern; zentralvenösen Kathetern; Venenkathetem oder Einlass- bzw. Auslass-Kathetern; Dilationsballons; Kathetern für die Angioplastie und die Biopsie; Kathetern, die für das Einbringen eines Stents, eines Propf- oder eines Kavafilters verwendet werden; Ballonkathetern oder anderen dehnbaren medizinischen Geräten; Endoskopen; Laryngoskopen; Trachealgeräten wie Endotrachealschläuchen; Atemgeräten und Trachealabsauggeräten; bronchoalveolaren Spülungskathetern; Kathetern, die bei der Koronarangioplastie verwendet werden; Führungsstäben und Einführern; Gefäßpfropfen; Schrittmacherteilen; Cochleaimplantaten; Zahnimplantatschläuchen für die Nahrungszufuhr; Dränageschläuchen; Führungsdrähten; Handschuhen; Stents und anderen Implantaten; extrakorporalen Blutschläuchen; Membranen, wie für die Dialyse; Blutfiltern; Geräten für die Kreislaufunterstützung; Verbandsmaterialien für die Wundpflege; Harnbeuteln; Stomabeuteln; Implantaten, die ein medizinisch wirksames Mittel enthalten, wie medizinisch wirksame Mitteln für Stents oder für Ballonoberflächen oder für Kontrazeptiva; Endoskopen, Laryngoskopen und Ernährungsschläuchen.

10. Medizinisches Gerät nach Anspruch 9 in der Form eines radioaktiven Stents, eines mit Medikamenten beschichteten Stents, eines bioresorbierbaren Stents und eines *healing-*Stents.

## Claims

1. Medical device having at least one coating comprising at least one polyurethaneurea
**characterized in that**
the polyurethaneurea is terminated with a copolymer unit comprising polyethylene oxide and polypropylene oxide.

2. Medical device according to Claim 1, **characterized in that** the polyurethaneurea comprises units which originate from at least one hydroxyl-containing polycarbonate.

3. Medical device according to Claim 1 or 2,
**characterized in that** the polyurethaneurea comprises units which originate from at least one aliphatic, cycloaliphatic or aromatic isocyanate.

4. Medical device according to any one of Claims 1 to 3, **characterized in that** the polyurethaneurea comprises units which originate from at least one diamine or amine alcohol.

5. Medical device according to one of Claims 1 to 4,
**characterized in that** the coating comprises a polyurethaneurea which is synthesized from
a) at least one polycarbonate polyol having an average molar weight between 400 g/mol and 6000 g/mol and a hydroxyl functionality of 1.7 to 2.3, or mixtures of such polycarbonate polyols;
b) at least one aliphatic, cycloaliphatic or aromatic polyisocyanate or mixtures of such polyisocyanates in an amount per mole of the polycarbonate polyol of 1.0 to 4.0 mol;
c) at least one monofunctional mixed polyoxyalkylene ether comprising polyethylene oxide and polypropylene oxide or a mixture of such polyethers, having an average molar weight between 500 g/mol and 5000 g/mol, in an amount per mole of the polycarbonate polyol of 0.01 to 0.5 mol;
d) at least one aliphatic or cycloaliphatic diamine or at least one amino alcohol, as so-called chain extenders, or mixtures of such compounds in an amount per mole of the polycarbonate polyol of 0.05 to 3.0 mol;
e) if desired, one or more short-chain aliphatic polyols having a molar weight between 62 g/mol and 500 g/mol, in an amount per mole of the polycarbonate polyol of 0.1 to 1.0 mol; and
f) if desired, amine- or OH-containing units which are located on, and cap, the polymer chain ends.

6. Process for producing a medical device having at least one coating, the coating being obtainable starting from a dispersion comprising at least one polyurethaneurea in accordance with the definition in any one of Claims 1 to 5.

7. Prodess according to Claim 6, **characterized in that** the coating starting from a dispersion of the polyurethaneurea is applied to the medical device by knifecoating, printing, transfer coating, spraying, spin coating or dipping.

8. Medical device obtainable according to Claim 6 or 7.

9. Medical device according to any one of Claims 1 to 5 or 8 in the form of contact lenses; cannulas; catheters, more particularly urological catheters such as urinary catheters or ureteral catheters; central venous catheters; venous catheters or inlet or outlet catheters; dilation balloons; catheters for angioplasty and biopsy; catheters used for introducing a stent, an embolism filter or a vena caval filter; balloon catheters or other expandable medical devices; endoscopes; laryngoscopes; tracheal devices such as endotracheal tubes, respirators and tracheal aspiration devices; bronchoalveolar lavage catheters; catheters used in coronary angioplasty; guide rods and insertion guides; vascular plugs; pacemaker components; cochlear implants; dental implant tubes for feeding; drainage tubes; guide wires; gloves; stents and other implants; extracorporeal blood lines; membranes, such as for dialysis; blood filters; devices for circulatory support; dressing materials for wound management; urine bags; stoma bags; implants which comprise a medically active agent, such as medically active agents for stents or for balloon surfaces or for contraceptives; endoscopes, laryngoscopes and feeding tubes.

10. Medical device according to Claim 9 in the form of a radioactive stent, a drug-coated stent, bioabsorbable stent or a *healing* stent.

## Revendications

1. Instrument médical avec au moins un revêtement, contenant au moins une polyuréthane-urée, **caractérisé en ce que** la polyuréthane-urée est terminée avec une unité copolymère en oxyde de polyéthylène et oxyde de polypropylène.

2. Instrument médical selon la revendication 1, **caractérisé en ce que** la polyuréthane-urée présente des unités, qui remontent à au moins un polycarbonate contenant des groupes hydroxyle.

3. Instrument médical selon la revendication 1 ou 2, **caractérisé en ce que** la polyuréthane-urée présente des unités, qui remontent à au moins un isocyanate aliphatique, cycloaliphatique ou aromatique.

4. Instrument médical selon l'une quelconque des revendications 1 à 3, **caractérisé en ce que** la polyuréthane-urée présente des unités, qui remontent à au moins une diamine ou un alcool aminé.

5. Instrument médical selon l'une quelconque des revendications 1 à 4, **caractérisé en ce que** le revêtement présente une polyuréthane-urée, qui est composée de:
a) au moins un polyol de polycarbonate ayant un poids moléculaire moyen compris entre 400 g/mole et 6000 g/mole et une fonctionnalité hydroxyle de 1,7 à 2,3 ou de mélanges de tels polyols de polycarbonate;
b) au moins un polyisocyanate aliphatique, cycloaliphatique ou aromatique ou des mélanges de tels polyisocyanates en une quantité de 1,0 à 4,0 mole par mole du polyol de polycarbonate;
c) au moins un éther de polyoxyalkylène mélangé monofonctionnel composé d'oxyde de polyéthylène et d'oxyde de polypropylène ou un mélange de tels polyéthers ayant un poids moléculaire moyen entre 500 g/mole et 5000 g/mole en une quantité de 0,01 à 0,5 mole par mole du polyol de polycarbonate;
d) au moins une diamine aliphatique ou cycloaliphatique ou au moins un alcool aminé comme allongeur de chaîne ou des mélanges de tels composés en une quantité de 0,05 à 3,0 moles par mole du polyol de polycarbonate;
e) éventuellement un ou plusieurs polyols aliphatiques à chaîne courte ayant un poids moléculaire entre 62 g/mole et 500 g/mole en une quantité de 0,1 à 1,0 mole par mole du polyol de polycarbonate; et
f) éventuellement des blocs contenant un groupe amino ou OH, qui se trouvent sur les extrémités de la chaîne polymère et terminent celles-ci.

6. Procédé de fabrication d'un instrument médical avec au moins un revêtement, dans lequel le revêtement peut être obtenu à partir d'une dispersion, contenant au moins une polyuréthane-urée selon la définition donnée dans l'une quelconque des revendications 1 à 5.

7. Procédé selon la revendication 6, **caractérisé en ce que** l'on applique le revêtement sur l'instrument médical à partir d'une dispersion de la polyuréthane-urée par raclage, impression, revêtement par transfert, pulvérisation, enduction centrifuge ou immersion.

8. Instrument médical, pouvant être obtenu selon la revendication 6 ou 7.

9. Instrument médical selon l'une quelconque des revendications 1 à 5 ou 8 sous la forme de lentilles de contact; canules; cathéters, en particulier cathéters urologiques ou cathéters de la vessie ou cathéters de l'uretère; cathéters veineux centraux; cathéters veineux ou cathéters d'entrée ou de sortie; ballonnets de dilatation; cathéters pour l'angioplastie et la biopsie; cathéters, qui sont utilisés pour la pose d'un stent, d'un filtre-tampon ou d'un filtre-cave; cathéters à ballonnet ou autres instruments médicaux extensibles; endoscopes; laryngoscopes; instruments de trachée comme des tuyaux pour intubation endotrachéale; appareils respiratoires et appareils d'aspiration trachéale; cathéters pour lavage bronchoalvéolaire; cathéters, qui sont utilisés pour l'angioplastie coronaire; tiges de guidage et guides; tampons de vaisseaux; pièces de stimulateur cardiaque; implants cochléaires; tuyaux d'implants dentaires pour l'apport d'aliments; tuyaux de drainage; fils de guidage; gants; stents et autres implants; tuyaux sanguins extracorporels; membranes, comme pour la dialyse; filtres sanguins; instruments pour soutenir la circulation du sang; matières de pansements pour le soin des blessures; poches à urée; poches buccales; implants, qui contiennent un agent médicalement actif, comme des agents médicalement actifs pour des stents ou pour des surfaces de ballonnets ou pour des contraceptifs; endoscopes, laryngoscopes et tubes d'alimentation.

10. Instrument médical selon la revendication 9 sous la forme d'un stent radioactif, d'un stent revêtu de médicaments, d'un stent biorésorbable et d'un stent de cicatrisation.
